# EUROPEAN PATENT APPLICATION

(11) **EP 4 218 561 A1**
(43) Date of publication of application: **02.08.2023**
(21) Application number: 21872193.4
(22) Date of filing: 09.09.2021
(51) Int. Cl.: A61B 5/055, A61B 10/00

(54) **DIAGNOSIS ASSISTANCE APPARATUS, DIAGNOSIS ASSISTANCE APPARATUS OPERATION METHOD, AND DIAGNOSIS ASSISTANCE APPARATUS OPERATION PROGRAM**

(30) Priority: 28.09.2020 JP 2020162680
(71) Applicant: FUJIFILM Corporation, Tokyo 106-8620 (JP)
(72) Inventor: WANG, Caihua, Ashigarakami-gun, Kanagawa 258-8577 (JP); LI, Yuanzhong, Ashigarakami-gun, Kanagawa 258-8577 (JP)
(74) Representative: Klunker IP Patentanwälte PartG mbB
(86) International application number: PCT/JP2021/033191
(87) International publication number: WO 2022/065062

(57) **Abstract**

A diagnosis support device includes a processor and a memory connected to or built in the processor. The processor is configured to perform non-linear registration processing of a target image which is a medical image to be analyzed and at least one representative image generated from a plurality of medical images, input at least one of transformation amount information of the target image obtained by the non-linear registration processing or a feature amount derived from the transformation amount information to a disease opinion derivation model, and output a disease opinion from the disease opinion derivation model.

## Description

### BACKGROUND OF THE INVENTION

### 1. Field of the Invention

A technique of the present disclosure relates to a diagnosis support device, an operation method of a diagnosis support device, and an operation program of a diagnosis support device.

### 2. Description of the Related Art

In a medical field, with a recent progress of artificial intelligence techniques, various techniques of inputting medical images to a machine learning model and outputting a disease opinion from the machine learning model have been proposed.

For example, <X. Long, L. Chen et al.: Prediction and classification of Alzheimer disease based on quantification of MRI deformation, 2017> describes a technique of performing non-linear registration processing of a medical image to be analyzed (hereinafter, abbreviated as a target image) and all the other medical images, acquiring transformation amount information of the target image with respect to all the other medical images, inputting a feature amount derived from the transformation amount information to a machine learning model, and outputting a disease opinion from the machine learning model. The transformation amount information is a set of three-dimensional movement vectors indicating movement of each pixel of the target image by the non-linear registration processing, and is also called a motion field.

### SUMMARY OF THE INVENTION

However, in the technique described in <X. Long, L. Chen et al.: Prediction and classification of Alzheimer disease based on quantification of MRI deformation, 2017>, it is necessary to perform non-linear registration processing of the target image and all the other medical images. As a result, it may take a long processing time to an extent that there is a practical problem.

An embodiment according to the technique of the present disclosure provides a diagnosis support device, an operation method of a diagnosis support device, and an operation program of a diagnosis support device capable of shortening a processing time.

According to an aspect of the present disclosure, there is provided a diagnosis support device including: a processor; and a memory connected to or built in the processor, in which the processor is configured to perform non-linear registration processing of a target image which is a medical image to be analyzed and at least one representative image generated from a plurality of medical images, and input at least one of transformation amount information of the target image obtained by the non-linear registration processing or a feature amount derived from the transformation amount information to a disease opinion derivation model, and output a disease opinion from the disease opinion derivation model.

Preferably, the representative image is prepared for each of a plurality of attribute groups according to attributes of a patient.

Preferably, the attribute is at least one of age or gender.

Preferably, the representative image is prepared for at least one of a plurality of opinion groups obtained by further dividing the attribute groups according to the opinion.

Preferably, the opinion is content as to whether or not the disease is progressed, and the opinion groups include a disease progression group in which the disease is progressed and a disease non-progression group in which the disease is not progressed.

Preferably, the representative image is generated by repeatedly performing a series of processing, which includes processing of generating a plurality of registration images corresponding to the plurality of medical images by performing non-linear registration processing of the plurality of medical images and a first temporary representative image and processing of generating a second temporary representative image from the plurality of registration images, until an instruction to adopt the second temporary representative image as the representative image is input.

Preferably, the medical image is an image in which a head of a patient appears, and the disease opinion derivation model is a model that outputs, as the opinion, a dementia opinion on the patient.

Preferably, the feature amount is a local atrophy rate derived by applying a Jacobi matrix to the transformation amount information.

According to another aspect of the present disclosure, there is provided an operation method of a diagnosis support device, the method including: performing non-linear registration processing of a target image which is a medical image to be analyzed and at least one representative image generated from a plurality of medical images; and inputting at least one of transformation amount information of the target image obtained by the non-linear registration processing or a feature amount derived from the transformation amount information to a disease opinion derivation model, and outputting a disease opinion from the disease opinion derivation model.

According to still another aspect of the present disclosure, there is provided an operation program of a diagnosis support device, the program causing a computer to execute a process including: performing non-linear registration processing of a target image which is a medical image to be analyzed and at least one representative image generated from a plurality of medical images; and inputting at least one of transformation amount information of the target image obtained by the non-linear registration processing or a feature amount derived from the transformation amount information to a disease opinion derivation model, and outputting a disease opinion from the disease opinion derivation model.

According to the technique of the present disclosure, it is possible to provide a diagnosis support device, an operation method of a diagnosis support device, and an operation program of a diagnosis support device capable of shortening a processing time.

### BRIEF DESCRIPTION OF THE DRAWINGS

Fig. 1 is a diagram illustrating a medical system including a diagnosis support device.
Fig. 2 is a block diagram illustrating a computer including the diagnosis support device.
Fig. 3 is a block diagram illustrating a processing unit of a CPU of the diagnosis support device.
Fig. 4 is a diagram illustrating processing of a normalization unit.
Fig. 5 is a diagram illustrating a representative image table.
Fig. 6 is a flowchart illustrating a method of generating a representative image.
Fig. 7 is a diagram illustrating a method of generating a representative image.
Fig. 8 is a diagram illustrating processing of a non-linear registration unit.
Fig. 9 is a diagram illustrating processing of the non-linear registration unit.
Fig. 10 is a diagram illustrating processing of a dementia opinion derivation unit.
Fig. 11 is a diagram illustrating an outline of processing in a learning phase of a dementia opinion derivation model.
Fig. 12 is a diagram illustrating a first display screen.
Fig. 13 is a diagram illustrating a second display screen.
Fig. 14 is a flowchart illustrating a processing procedure of the diagnosis support device.
Fig. 15 is a diagram illustrating a form in which a normalization target image and one piece of transformation amount information are input to a dementia opinion derivation model and dementia opinion information is output from the dementia opinion derivation model.
Fig. 16 is a diagram illustrating a second embodiment in which a local atrophy rate is derived from transformation amount information.
Fig. 17 is a diagram illustrating another example of dementia opinion information.
Fig. 18 is a diagram illustrating still another example of dementia opinion information.

### DESCRIPTION OF THE PREFERRED EMBODIMENTS

### [First Embodiment]

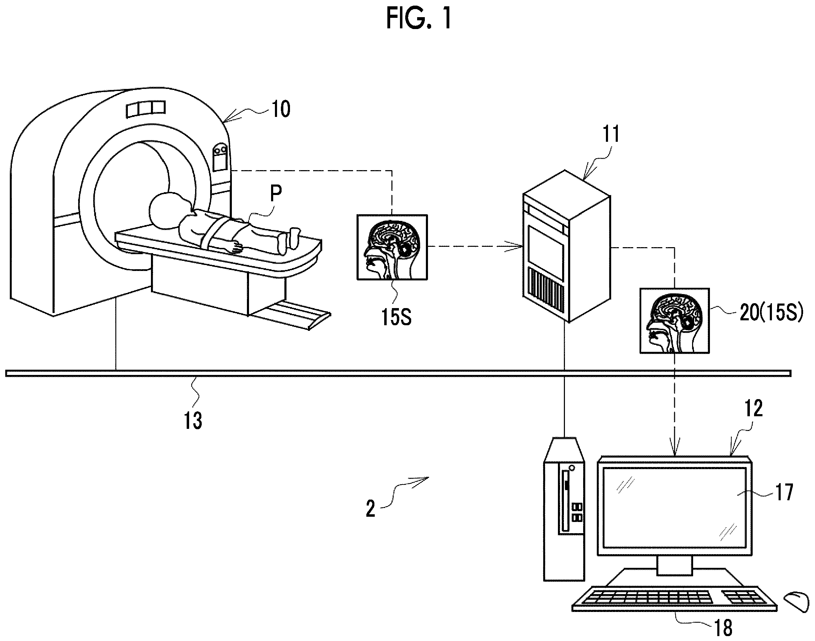

As illustrated in Fig. 1 as an example, a medical system 2 includes a magnetic resonance imaging (MRI) apparatus 10, a picture archiving and communication system (PACS) server 11, and a diagnosis support device 12. The MRI apparatus 10, the PACS server 11, and the diagnosis support device 12 are connected to a local area network (LAN) 13 provided in a medical facility, and can communicate with each other via the LAN 13.

The MRI apparatus 10 images a head of a patient P and outputs a head MRI image 15. The head MRI image 15 is voxel data representing a three-dimensional shape of the head of the patient P. In Fig. 1, a head MRI image 15S having a sagittal cross section is illustrated. The MRI apparatus 10 transmits the head MRI image 15 to the PACS server 11. The PACS server 11 stores and manages the head MRI image 15 from the MRI apparatus 10. The head MRI image 15 is an example of a "medical image" according to the technique of the present disclosure.

The diagnosis support device 12 is, for example, a desktop personal computer, and includes a display 17 and an input device 18. The input device 18 is a keyboard, a mouse, a touch panel, a microphone, or the like. A doctor transmits a distribution request of the head MRI image 15 of the patient P to the PACS server 11 by operating the input device 18. The PACS server 11 searches for the head MRI image 15 of the patient P that is requested to be distributed, and distributes the head MRI image 15 to the diagnosis support device 12. The diagnosis support device 12 displays the head MRI image 15 distributed from the PACS server 11 on the display 17. The doctor diagnoses dementia on the patient P by observing a brain of the patient P appearing in the head MRI image 15. In the following, the head MRI image 15 for diagnosing dementia will be referred to as a target image 20 in order to distinguish the head MRI image 15 from other head MRI images 15. The dementia is an example of a "disease" according to the technique of the present disclosure. Further, in Fig. 1, only one MRI apparatus 10 and one diagnosis support device 12 are illustrated. On the other hand, a plurality of MRI apparatuses 10 and a plurality of diagnosis support devices 12 may be provided.

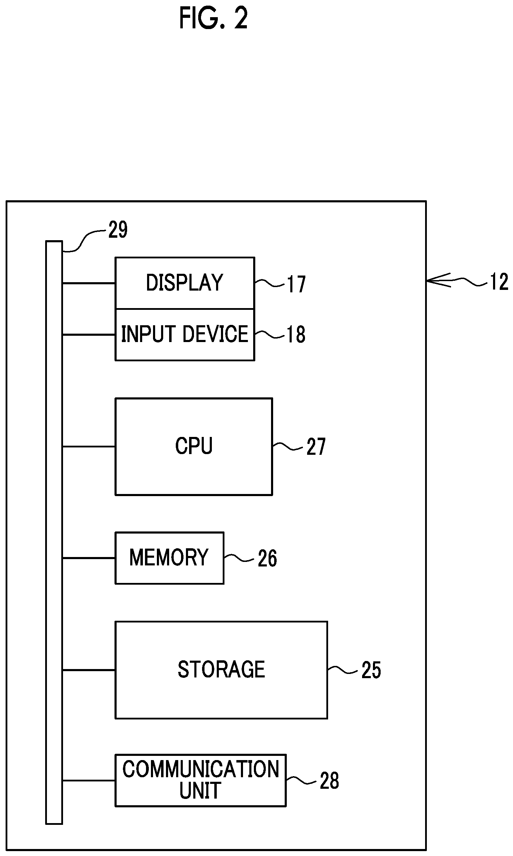

As illustrated in Fig. 2 as an example, a computer including the diagnosis support device 12 includes a storage 25, a memory 26, a central processing unit (CPU) 27, and a communication unit 28, in addition to the display 17 and the input device 18. The components are connected to each other via a bus line 29. The CPU 27 is an example of a "processor" according to the technique of the present disclosure.

The storage 25 is a hard disk drive that is built in the computer including the diagnosis support device 12 or is connected via a cable or a network. Alternatively, the storage 25 is a disk array in which a plurality of hard disk drives are connected in series. The storage 25 stores a control program such as an operating system, various application programs, and various data associated with the programs. A solid state drive may be used instead of the hard disk drive.

The memory 26 is a work memory which is necessary to execute processing by the CPU 27. The CPU 27 loads the program stored in the storage 25 into the memory 26, and executes processing according to the program. Thereby, the CPU 27 collectively controls each unit of the computer. The communication unit 28 controls transmission of various types of information to an external apparatus such as the PACS server 11. The memory 26 may be built in the CPU 27.

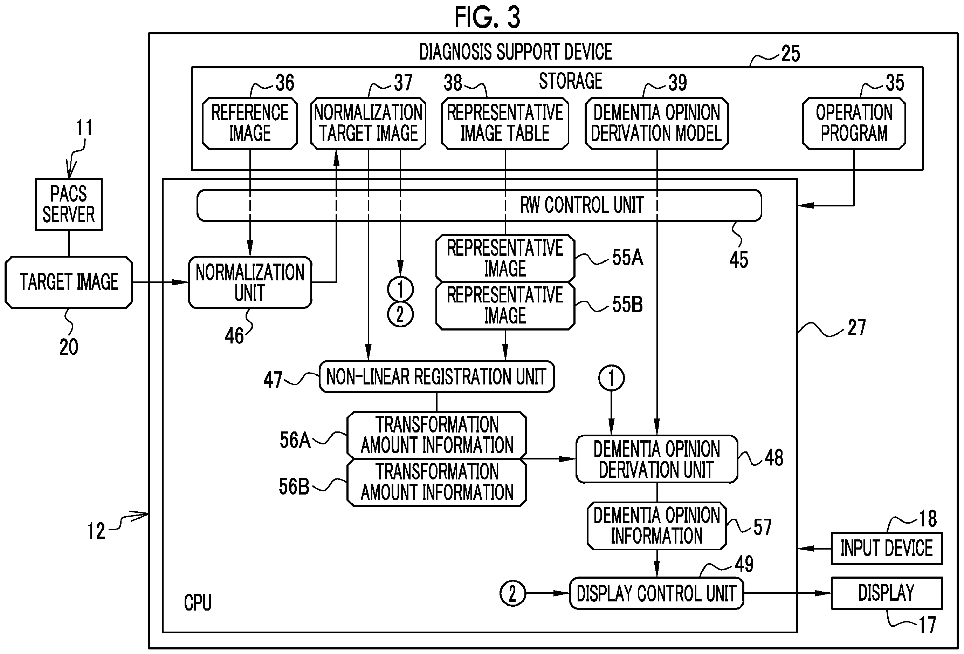

As illustrated in Fig. 3 as an example, an operation program 35 is stored in the storage 25 of the diagnosis support device 12. The operation program 35 is an application program for causing the computer to function as the diagnosis support device 12. That is, the operation program 35 is an example of "the operation program of the diagnosis support device" according to the technique of the present disclosure. The storage 25 also stores a reference image 36, a normalization target image 37, a representative image table 38, and a dementia opinion derivation model 39.

In a case where the operation program 35 is started, the CPU 27 of the computer including the diagnosis support device 12 functions as a read/write (hereinafter, abbreviated as RW) control unit 45, a normalization unit 46, a non-linear registration unit 47, a dementia opinion derivation unit 48, and a display control unit 49, in cooperation with the memory 26 and the like.

The RW control unit 45 controls storing of various types of data in the storage 25 and reading of various types of data in the storage 25. For example, the RW control unit 45 reads the reference image 36 from the storage 25, and outputs the read reference image 36 to the normalization unit 46. Further, the RW control unit 45 receives the normalization target image 37 from the normalization unit 46, and stores the received normalization target image 37 in the storage 25.

The RW control unit 45 reads the normalization target image 37 from the storage 25, and outputs the read normalization target image 37 to the non-linear registration unit 47, the dementia opinion derivation unit 48, and the display control unit 49. In addition, the RW control unit 45 reads two representative images 55A and 55B corresponding to the normalization target image 37 from the representative image table 38 of the storage 25, and outputs the read representative images 55A and 55B to the non-linear registration unit 47. The representative images 55A and 55B are images representing a plurality of head MRI images 15. Further, the RW control unit 45 reads the dementia opinion derivation model 39 from the storage 25, and outputs the read dementia opinion derivation model 39 to the dementia opinion derivation unit 48.

The normalization unit 46 performs normalization processing of matching the target image 20 from the PACS server 11 with the reference image 36, and sets the target image 20 as the normalization target image 37. The normalization unit 46 outputs the normalization target image 37 to the RW control unit 45.

The reference image 36 is a head MRI image in which a brain having a reference shape, a reference size, and a reference shade (pixel value) appears. The reference image 36 is, for example, an image generated by averaging head MRI images 15 of a plurality of healthy persons, or an image generated by computer graphics.

The non-linear registration unit 47 performs non-linear registration processing of the normalization target image 37 and the two representative images 55A and 55B. The non-linear registration unit 47 outputs transformation amount information 56A, which is a result of the non-linear registration processing of the normalization target image 37 and the representative image 55A, to the dementia opinion derivation unit 48. In addition, the non-linear registration unit 47 outputs transformation amount information 56B, which is a result of the non-linear registration processing of the normalization target image 37 and the representative image 55B, to the dementia opinion derivation unit 48. In the following description, in a case where it is not necessary to distinguish between the representative images 55A and 55B and between the transformation amount information 56A and 56B, the representative images 55A and 55B are collectively referred to as the representative image 55, and the transformation amount information 56A and 56B are collectively referred to as the transformation amount information 56.

The dementia opinion derivation unit 48 inputs the normalization target image 37 and the transformation amount information 56 into the dementia opinion derivation model 39. In addition, dementia opinion information 57 representing a dementia opinion is output from the dementia opinion derivation model 39. The dementia opinion derivation model 39 is configured, for example, by a method of a neural network. The dementia opinion derivation unit 48 outputs the dementia opinion information 57 to the display control unit 49. The dementia opinion derivation model 39 is an example of a "disease opinion derivation model" according to the technique of the present disclosure.

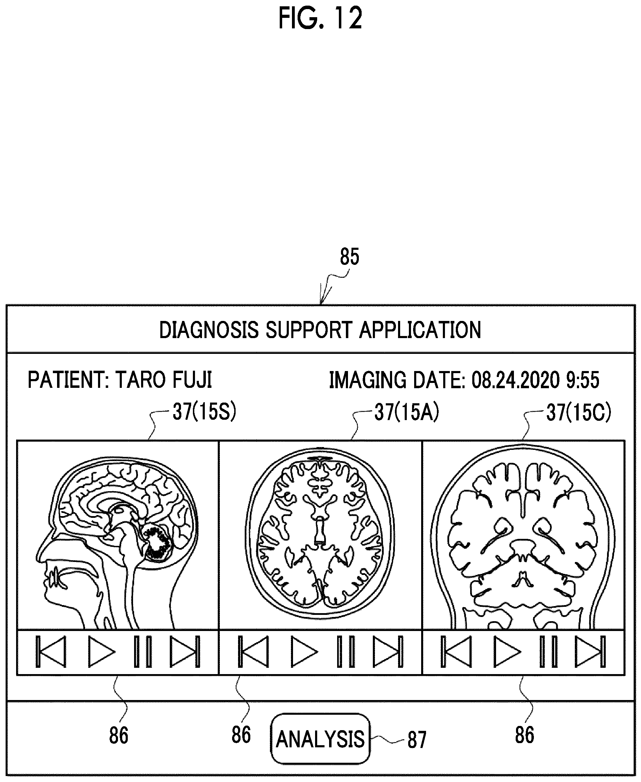

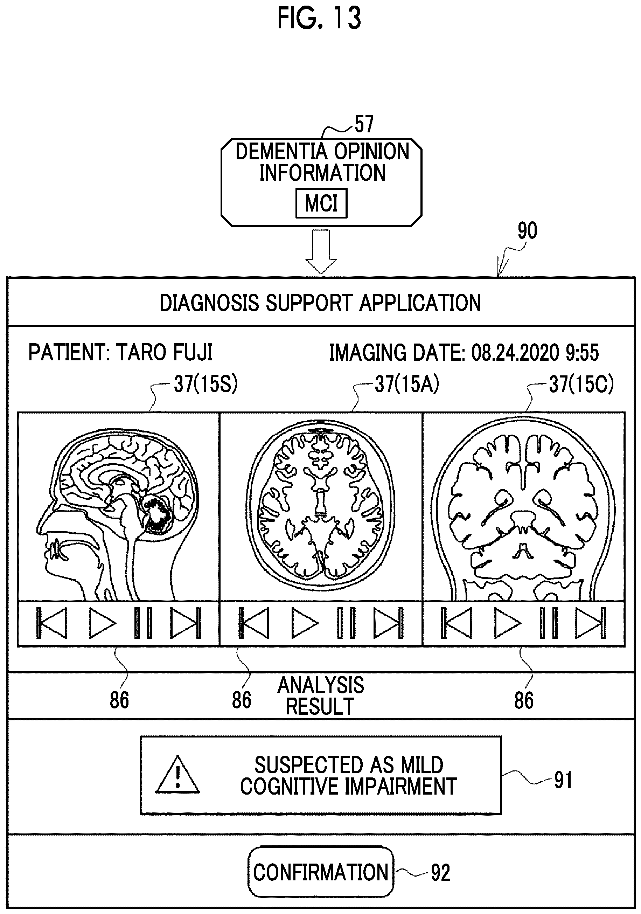

The display control unit 49 controls a display of various screens on the display 17. The various screens include a first display screen 85 (refer to Fig. 12) for instructing analysis by the dementia opinion derivation model 39, a second display screen 90 (refer to Fig. 13) for displaying the dementia opinion information 57, and the like.

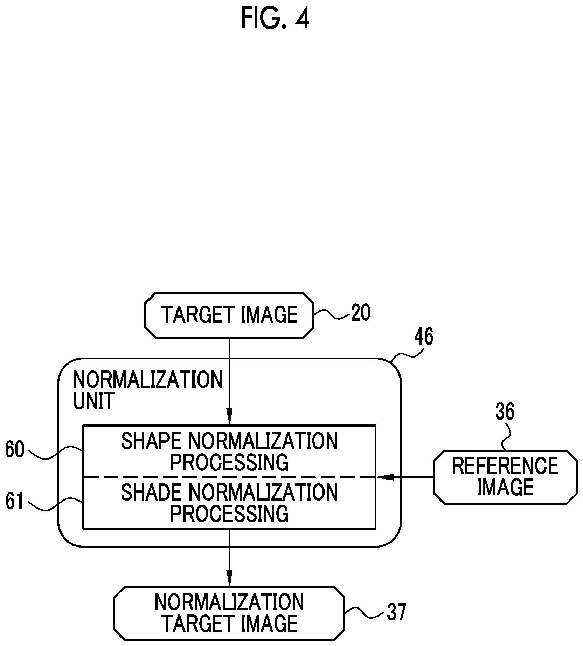

As illustrated in Fig. 4 as an example, the normalization unit 46 performs, as normalization processing, shape normalization processing 60 and shade normalization processing 61 on the target image 20. The shape normalization processing 60 is processing of extracting, for example, landmarks serving as references for registration from the target image 20 and the reference image 36, and performing parallel displacement, rotation, and/or enlargement/reduction of the target image 20 in accordance with the reference image 36 such that a correlation between the landmark of the target image 20 and the landmark of the reference image 36 is maximized. The shade normalization processing 61 is, for example, processing of correcting a shade histogram of the target image 20 in accordance with a shade histogram of the reference image 36.

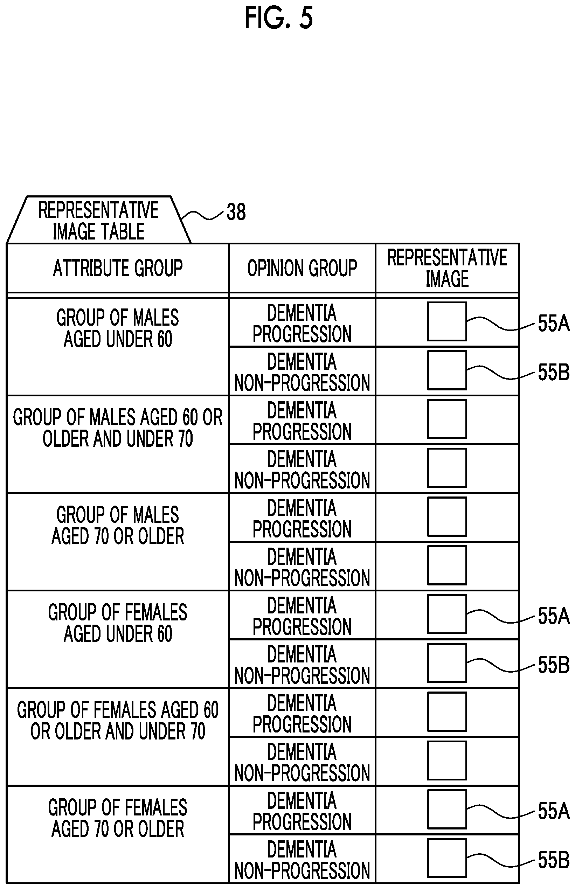

As illustrated in Fig. 5 as an example, in the representative image table 38, the two representative images 55A and 55B are registered for each attribute group. The attribute group is a group divided by age and gender, which are attributes of the patient P. Specifically, the attribute group includes total 6 groups of a group of males aged under 60, a group of males aged 60 or older and under 70, a group of males aged 70 or older, a group of females aged under 60, a group of females aged 60 or older and under 70, and a group of females aged 70 or older. The attribute group is not limited thereto, and for example, a group of males aged 80 or older, a group of females aged 80 or older, and the like may be added.

The attribute group is further divided into two opinion groups according to the opinion. The opinion is content as to whether or not dementia is progressed. The opinion groups include, for example, two groups of a dementia progression group in which dementia is progressed more now than two years ago and a dementia non-progression group in which dementia is not progressed from two years ago to the present. In each attribute group, the representative image 55A is registered in the dementia progression group, and the representative image 55B is registered in the dementia non-progression group. The RW control unit 45 reads the representative images 55A and 55B registered in the attribute group having the same attributes as the patient P of the target image 20 from the representative image table 38, and outputs the read representative images 55A and 55B to the non-linear registration unit 47.

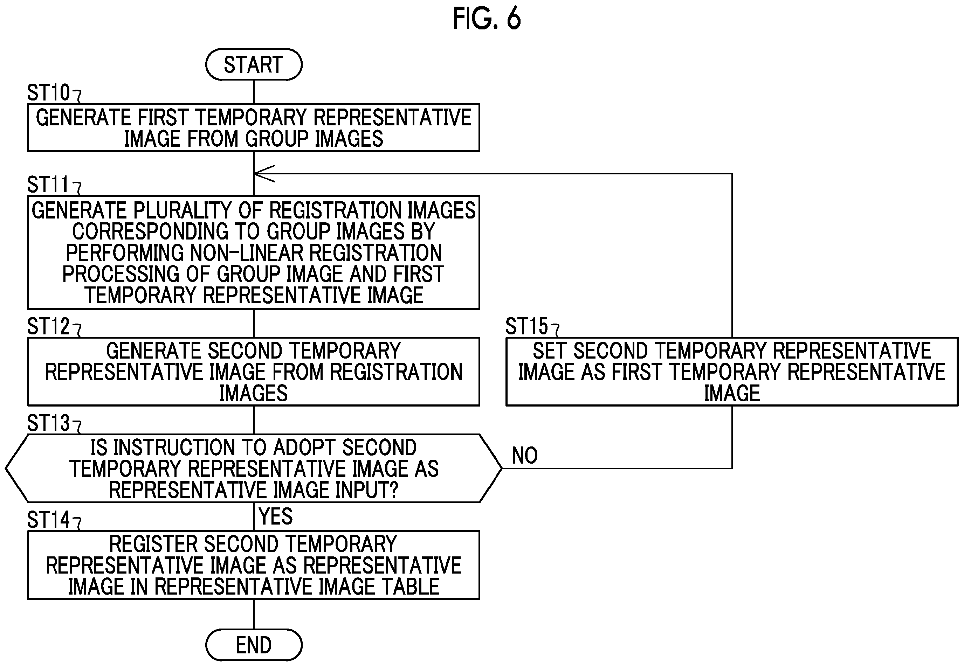

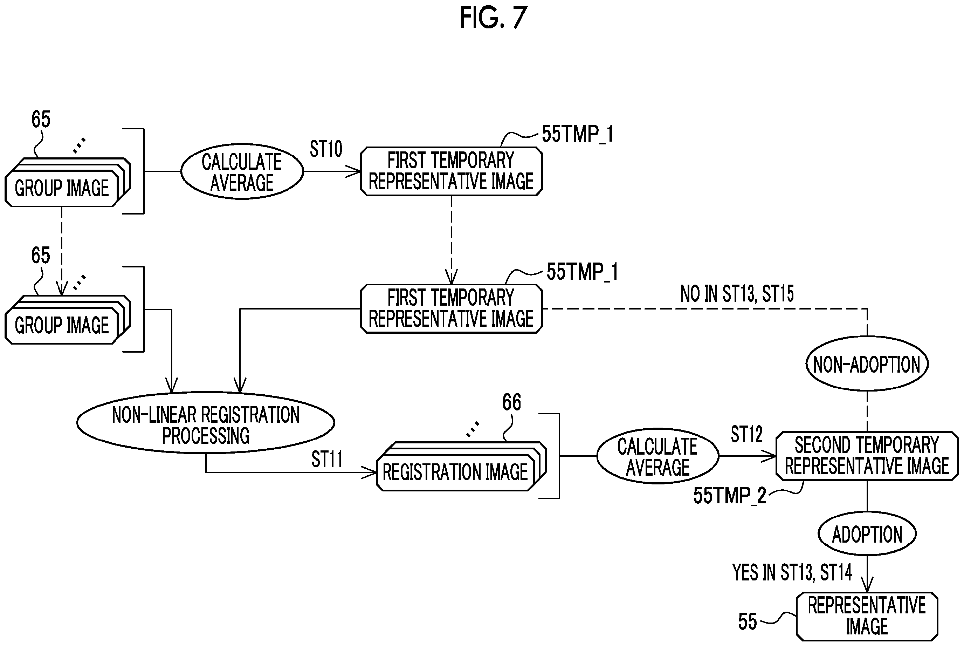

Here, a method of generating the representative image 55 will be described below with reference to a flowchart of Fig. 6 and Fig. 7.

A device that generates the representative image 55 classifies, as preprocessing, a plurality of head MRI images 15 provided by a plurality of medical facilities into the above-described six attribute groups. Further, the head MRI images 15 of each attribute group are also classified into the above-described two opinion groups. Thus, the head MRI images 15 classified into each group are referred to as group images 65. It is assumed that the head MRI images 15 used for generating the representative image 55 are images on which the normalization processing is performed.

An average value of a pixel value of each pixel of each of a plurality of group images 65 belonging to a certain group is calculated, and a first temporary representative image 55TMP_1 in which the calculated average value is set as a pixel value of each pixel is generated (step ST10). In addition, by performing the non-linear registration processing of each of the plurality of group images 65 and the first temporary representative image 55TMP_1, a plurality of registration images 66 corresponding to the plurality of group images 65 are generated (step ST11). Subsequently, in the same manner as in step ST10, an average value of a pixel value of each pixel of each of the plurality of registration images 66 is calculated, and a second temporary representative image 55TMP_2 in which the calculated average value is set as a pixel value of each pixel is generated (step ST12). A median value of a pixel value of each pixel of the group image 65 or the registration image 66 may be derived instead of an average value of a pixel value of each pixel of the group image 65 or the registration image 66. In this case, a first temporary representative image 55TMP_1 or a second temporary representative image 55TMP _2 in which the derived median value is set as a pixel value of each pixel may be generated.

The generated second temporary representative image 55TMP_2 is displayed for a developer of the operation program 35 via the display. In addition, an instruction as to whether or not to adopt the second temporary representative image 55TMP_2 as the representative image 55 is input by the developer.

In a case where an instruction to adopt the second temporary representative image 55TMP_2 as the representative image 55 is input by the developer (YES in step ST13), the second temporary representative image 55TMP_2 is registered as the representative image 55 in the representative image table 38 (step ST14). On the other hand, in a case where an instruction not to adopt the second temporary representative image 55TMP_2 as the representative image 55 is input (NO in step ST13), the second temporary representative image 55TMP_2 is set as the first temporary representative image 55TMP _1 (step ST15), and processing of step ST11 and step ST12 is repeated again. By performing such processing on the group image 65 of each group, the representative image 55 of each group is generated. The device that generates the representative image 55 is a device operated by the developer at a development stage of the operation program 35, and is, for example, a device different from the diagnosis support device 12. Of course, the diagnosis support device 12 may generate the representative image 55. The representative image 55 may be periodically updated.

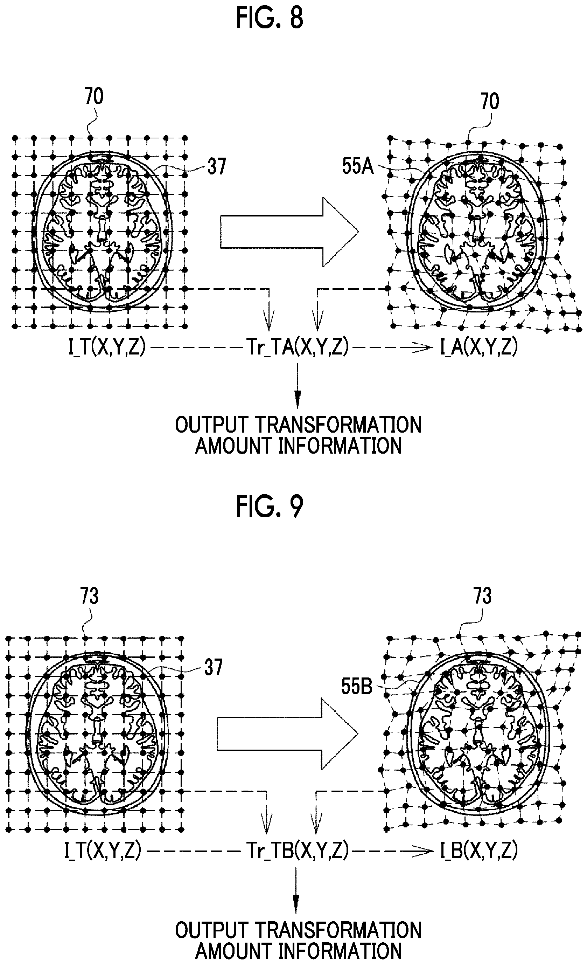

As illustrated in Fig. 8 as an example, in a case where the normalization target image 37 (denoted as I_T (X, Y, Z)) is non-linearly registered with the representative image 55A (denoted as I_A (X, Y, Z)), the non-linear registration unit 47 sets, on the normalization target image 37, a plurality of control points 70 which are arranged in a grid pattern at equal intervals. In addition, the non-linear registration unit 47 moves each control point 70 to a position at which a local similarity between the normalization target image 37 and the representative image 55A increases. The non-linear registration unit 47 derives, from a movement amount of each control point 70, a transformation amount Tr_TA (X, Y, Z) of each pixel in a case where the normalization target image 37 is registered with the representative image 55A, by using an interpolation approximation curve such as a B-Spline curve. The non-linear registration unit 47 outputs the derived transformation amount Tr_TA (X, Y, Z) as transformation amount information 56A.

In addition, as illustrated in Fig. 9 as an example, in a case where the normalization target image 37 is non-linearly registered with the representative image 55B (denoted as I_B (X, Y, Z)), the non-linear registration unit 47 sets, on the normalization target image 37, control points 73 similar to the control points 70. In addition, the non-linear registration unit 47 moves each control point 73 as in the case of Fig. 8. As in the case of Fig. 8, the non-linear registration unit 47 derives, from a movement amount of each control point 73, a transformation amount Tr_TB (X, Y, Z) of each pixel in a case where the normalization target image 37 is registered with the representative image 55B. The non-linear registration unit 47 outputs the derived transformation amount Tr_TB (X, Y, Z) as transformation amount information 56B. In the following description, (X, Y, Z) may be omitted. In addition, Fig. 8 and Fig. 9 illustrate a state where the control points 70 and 73 are set in a two-dimensional shape on the normalization target image 37 having an axial cross section. On the other hand, the control points 70 and 73 are actually set in a three-dimensional shape.

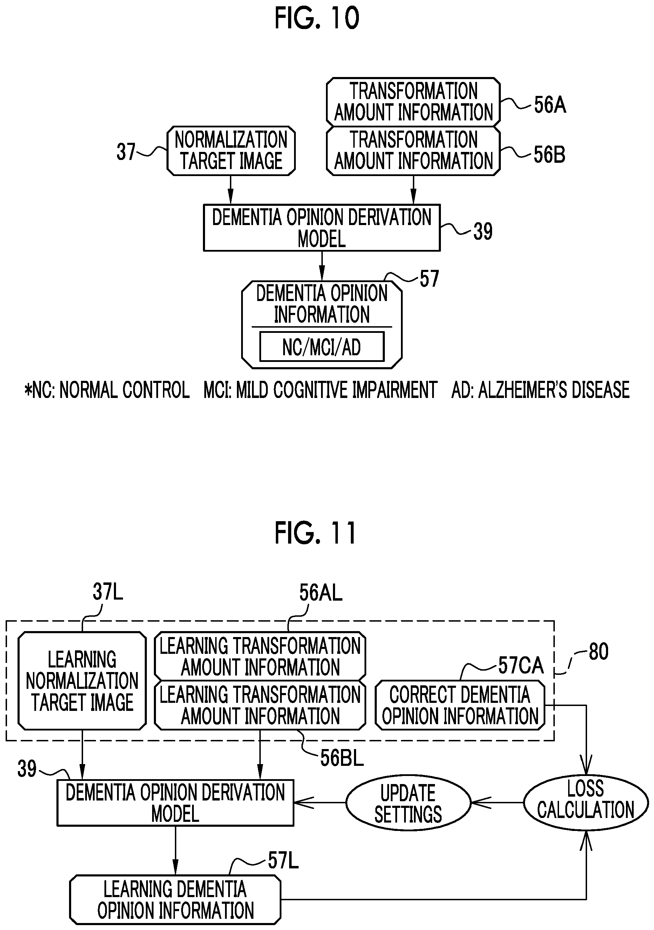

As illustrated in Fig. 10 as an example, in a case where the normalization target image 37 and the transformation amount information 56 are input, the dementia opinion information 57 is output from the dementia opinion derivation model 39. The dementia opinion information 57 is any one of normal control (NC), mild cognitive impairment (MCI), and Alzheimer's disease (AD).

Fig. 11 illustrates an example of an outline of processing in a learning phase of the dementia opinion derivation model 39. The dementia opinion derivation model 39 is trained by inputting learning data 80. The learning data 80 is a set of a learning normalization target image 37L, pieces of learning transformation amount information 56AL and 56BL, and correct dementia opinion information 57CA corresponding to the learning normalization target image 37L and the pieces of learning transformation amount information 56AL and 56BL. The learning normalization target image 37L is obtained by performing normalization processing on a certain head MRI image 15. The learning transformation amount information 56AL is obtained by performing non-linear registration processing on the learning normalization target image 37L and the representative image 55A. The learning transformation amount information 56BL is obtained by performing non-linear registration processing on the learning normalization target image 37L and the representative image 55B. The correct dementia opinion information 57CA is a result obtained by actually diagnosing a dementia opinion on the learning normalization target image 37L by a doctor.

In the learning phase, the learning normalization target image 37L and the pieces of learning transformation amount information 56AL and 56BL are input to the dementia opinion derivation model 39. The dementia opinion derivation model 39 outputs learning dementia opinion information 57L corresponding to the learning normalization target image 37L and the pieces of learning transformation amount information 56AL and 56BL. A loss calculation of the dementia opinion derivation model 39 using a loss function is performed based on the learning dementia opinion information 57L and the correct dementia opinion information 57CA. In addition, update settings of various coefficients of the dementia opinion derivation model 39 are performed according to a result of the loss calculation, and the dementia opinion derivation model 39 is updated according to the update settings.

In the learning phase of the dementia opinion derivation model 39, while exchanging the learning data 80, a series of pieces of processing, which includes inputting of the learning normalization target image 37L and the pieces of learning transformation amount information 56AL and 56BL to the dementia opinion derivation model 39, outputting of the learning dementia opinion information 57L from the dementia opinion derivation model 39, the loss calculation, the update settings, and updating of the dementia opinion derivation model 39, is repeatedly performed. The repetition of the series of pieces of processing is ended in a case where prediction accuracy of the learning dementia opinion information 57L with respect to the correct dementia opinion information 57CA reaches a predetermined set level. The dementia opinion derivation model 39 of which the prediction accuracy reaches the set level in this way is stored as a trained model in the storage 25, and is used in the dementia opinion derivation unit 48.

Fig. 12 illustrates an example of a first display screen 85 for instructing analysis by the dementia opinion derivation model 39. On the first display screen 85, the normalization image of the head MRI image 15 of the patient P for dementia diagnosis, that is, the normalization target image 37 is displayed. The normalization target image 37 includes a head MRI image 15S having a sagittal cross section, a head MRI image 15A having an axial cross section, and a head MRI image 15C having a coronal cross section. A button group 86 for switching the display is provided in a lower portion of each of the head MRI images 15S, 15A, and 15C.

On the first display screen 85, an analysis button 87 is provided. A doctor selects the analysis button 87 in a case where the doctor wants to perform analysis using the dementia opinion derivation model 39. Thereby, the CPU 27 receives an instruction for analysis using the dementia opinion derivation model 39.

Fig. 13 illustrates an example of a second display screen 90 that displays the dementia opinion information 57 obtained as a result of analysis by the dementia opinion derivation model 39. On the second display screen 90, a message 91 according to the dementia opinion information 57 is displayed. Fig. 13 illustrates an example in which the dementia opinion information 57 is mild cognitive impairment (MCI) and "suspected as mild cognitive impairment" is displayed as the message 91. In a case where a confirmation button 92 is selected, the display control unit 49 turns off the display of the message 91, and returns the second display screen 90 to the first display screen 85.

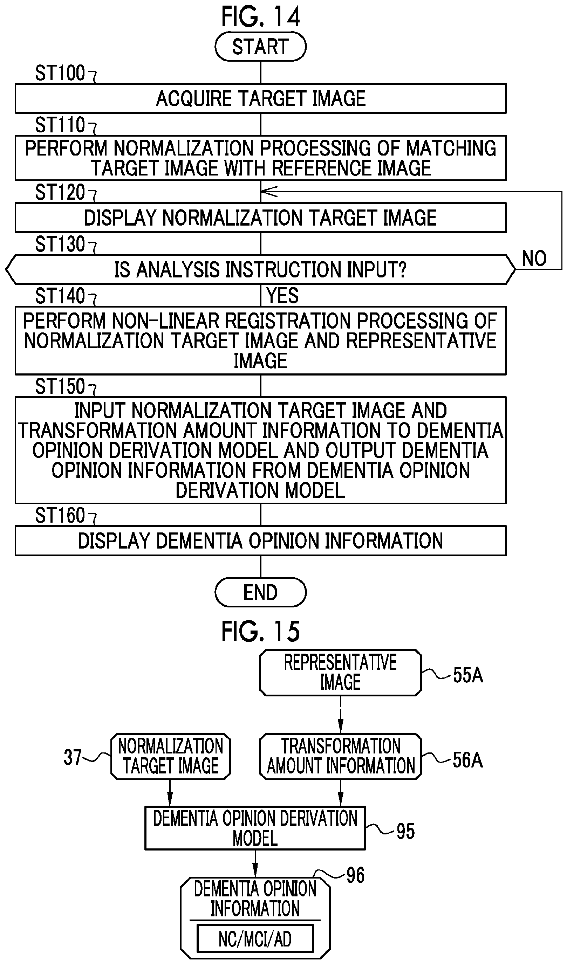

Next, an operation according to the configuration will be described with reference to a flowchart illustrated in Fig. 14. First, in a case where the operation program 35 is started in the diagnosis support device 12, as illustrated in Fig. 3, the CPU 27 of the diagnosis support device 12 functions as the RW control unit 45, the normalization unit 46, the non-linear registration unit 47, the dementia opinion derivation unit 48, and the display control unit 49.

First, the normalization unit 46 receives the target image 20 from the PACS server 11 (step ST100). In addition, as illustrated in Fig. 4, the normalization unit 46 performs normalization processing (shape normalization processing 60 and shade normalization processing 61) of matching the target image 20 with the reference image 36 (step ST110). Thereby, the target image 20 is set as the normalization target image 37. The normalization target image 37 is output from the normalization unit 46 to the RW control unit 45, and is stored in the storage 25 by the RW control unit 45.

The normalization target image 37 is read from the storage 25 by the RW control unit 45, and is output from the RW control unit 45 to the display control unit 49. In addition, under a control of the display control unit 49, the first display screen 85 illustrated in Fig. 12 is displayed on the display 17 (step ST120). In a case where the analysis button 87 is selected on the first display screen 85, an instruction for analysis by the dementia opinion derivation model 39 is received by the CPU 27 (YES in step ST130).

The normalization target image 37 is read from the storage 25 by the RW control unit 45, and is output to the non-linear registration unit 47 and the dementia opinion derivation unit 48. Further, the RW control unit 45 reads the representative images 55A and 55B corresponding to the normalization target image 37 from the storage 25, and outputs the representative images 55A and 55B to the non-linear registration unit 47.

In the non-linear registration unit 47, as illustrated in Fig. 8, non-linear registration processing of non-linearly registering the normalization target image 37 with the representative image 55A is performed. In addition, as illustrated in Fig. 9, non-linear registration processing of non-linearly registering the normalization target image 37 with the representative image 55B is performed (step ST140). The transformation amounts Tr_TA and Tr_TB derived by the non-linear registration processing are output from the non-linear registration unit 47 to the dementia opinion derivation unit 48, as pieces of transformation amount information 56A and 56B.

As illustrated in Fig. 10, in the dementia opinion derivation unit 48, the normalization target image 37 and the pieces of transformation amount information 56A and 56B are input to the dementia opinion derivation model 39. Thereby, the dementia opinion information 57 is output from the dementia opinion derivation model 39 (step ST150). The dementia opinion information 57 is output from the dementia opinion derivation unit 48 to the display control unit 49.

Under a control of the display control unit 49, the second display screen 90 illustrated in Fig. 13 is displayed on the display 17 (step ST160). A doctor confirms the dementia opinion information 57 via the message 91 on the second display screen 90.

As described above, the CPU 27 of the diagnosis support device 12 includes the non-linear registration unit 47 and the dementia opinion derivation unit 48. The non-linear registration unit 47 performs non-linear registration processing of the normalization target image 37 and the representative images 55A and 55B. The dementia opinion derivation unit 48 inputs the pieces of transformation amount information 56A and 56B of the normalization target image 37 that are obtained by the non-linear registration processing to the dementia opinion derivation model 39, and outputs the dementia opinion information 57 from the dementia opinion derivation model 39. Therefore, as compared with the technique in the related art in which non-linear registration processing needs to be performed on the normalization target image 37 and all the other head MRI images 15, it is possible to shorten a processing time.

As illustrated in Fig. 5, the representative image 55 is prepared for each of the plurality of attribute groups according to the attributes of the patient P. Therefore, the non-linear registration processing can be performed using the representative image 55 that matches the attributes of the patient P. As a result, it is possible to output appropriate dementia opinion information 57.

The attributes are age and gender. The brain naturally atrophies with age regardless of dementia. Therefore, in a case where age is included in the attributes, it is possible to diagnose dementia in consideration of atrophy due to aging. In addition, a degree of development of dementia, a degree of progression of dementia, and the like vary depending on gender. Therefore, in a case where gender is included in the attributes, it is possible to diagnose dementia in consideration of differences due to gender. In this example, both age and gender are adopted as the attributes. On the other hand, the attribute may be any one of age and gender. In addition, a past medical history of the patient P, a region where the patient P lives, and the like may be used as the attributes.

The representative images 55 are prepared for each of a plurality of opinion groups obtained by further dividing the attribute groups according to the opinion. Therefore, pieces of transformation amount information 56A and 56B for each opinion group can be obtained and set as the input data of the dementia opinion derivation model 39. Thereby, as compared with a case where the attribute groups are grouped without being divided into opinion groups, it is possible to improve reliability of the dementia opinion information 57.

As illustrated in Fig. 15, only the representative image 55A of the representative images 55A and 55B is prepared, and only the non-linear registration processing of the normalization target image 37 and the representative image 55A is performed. Thus, the transformation amount information 56A is output. In addition, only the normalization target image 37 and the transformation amount information 56A may be input to the dementia opinion derivation model 95, and the dementia opinion information 96 may be output from the dementia opinion derivation model 95. The dementia opinion information 96 is any one of normal control, mild cognitive impairment, or Alzheimer's disease, similarly to the dementia opinion information 57. Although not illustrated, on the contrary, only the representative image 55B may be prepared. That is, the representative image 55 does not have to be prepared for all of the plurality of opinion groups, and may be prepared for at least one of the plurality of opinion groups.

The opinion is content as to whether or not dementia is progressed, and the opinion groups include a dementia progression group in which dementia is progressed and a dementia non-progression group in which dementia is not progressed. Therefore, it is possible to obtain the transformation amount information 56 which is more useful for diagnosing dementia. Thereby, it is possible to further improve the prediction accuracy of the dementia opinion information 57.

As illustrated in Fig. 6 and Fig. 7, the representative image 55 is generated by repeatedly performing a series of processing until an instruction to adopt the second temporary representative image 55TMP_2 as the representative image 55 is input, the series of processing including processing of generating the plurality of registration images 66 corresponding to the plurality of group images 65 by performing the non-linear registration processing of the plurality of group images 65 and the first temporary representative image 55TMP_1 and processing of generating the second temporary representative image 55TMP_2 from the plurality of registration images 66. Therefore, it is possible to generate a relatively high quality representative image 55 without blurriness.

The dementia has become a social problem with the advent of an aging society in recent years. Therefore, it can be said that the present embodiment in which a medical image is the head MRI image 15 and a disease opinion derivation model is the dementia opinion derivation model 39 which outputs the dementia opinion information 57 is a form matching with the current social problem. In addition, the transformation amount, such as a degree of atrophy of hippocampus, parahippocampal gyrus, amygdala, or the like, is particularly important for the dementia opinion. The present embodiment uses the transformation amount information 56 of the normalization target image 37 that is obtained by the non-linear registration processing of the normalization target image 37 and the representative image 55. Therefore, it can be said that the present embodiment is more suitable for diagnosing dementia.

### [Second Embodiment]

The input data to the dementia opinion derivation model is not limited to the transformation amount information 56. A feature amount derived from the transformation amount information 56 may be used as the input data.

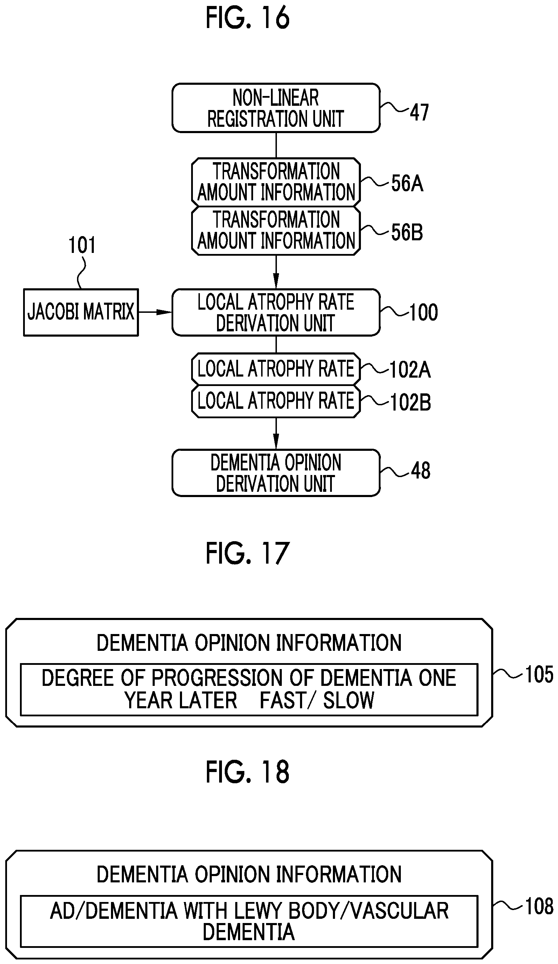

As illustrated in Fig. 16 as an example, in the second embodiment, a local atrophy rate derivation unit 100 is provided between the non-linear registration unit 47 and the dementia opinion derivation unit 48. The local atrophy rate derivation unit 100 derives a local atrophy rate 102A according to the transformation amount information 56A and a local atrophy rate 102B according to the transformation amount information 56B by applying a Jacobi matrix 101 to the transformation amount information 56A and the transformation amount information 56B from the non-linear registration unit 47. The local atrophy rate derivation unit 100 outputs the derived local atrophy rates 102A and 102B to the dementia opinion derivation unit 48. The dementia opinion derivation unit 48 provides, as the input data, the local atrophy rates 102A and 102B to the dementia opinion derivation model. The local atrophy rates 102A and 102B are an example of "feature amounts" according to the technique of the present disclosure.

As described above, in the second embodiment, the local atrophy rates 102A and 102B, which are the feature amounts derived from the transformation amount information 56, are input to the dementia opinion derivation model. The input data is simplified as compared with the transformation amount information 56, and thus the dementia opinion derivation model can also have a relatively simple configuration.

The feature amount is not limited to the local atrophy rates 102A and 102B. A representative value such as an average value or a maximum value of the transformation amount Tr_TA and the transformation amount Tr_TB may be used as the feature amount. In addition, as in a form illustrated in Fig. 15, instead of both the local atrophy rates 102A and 102B, any one of the local atrophy rates 102A and 102B may be derived and used as the input data of the dementia opinion derivation model. Both the transformation amount information 56 and the local atrophy rate 102 may be used as the input data of the dementia opinion derivation model.

The dementia opinion information 57 is not limited to the content illustrated in Fig. 10 (normal control/mild cognitive impairment/Alzheimer's disease). For example, as in the dementia opinion information 105 illustrated in Fig. 17, the dementia opinion information may indicate whether a degree of progression of dementia of the patient P one year later is fast or slow. Alternatively, as in the dementia opinion information 108 illustrated in Fig. 18, the dementia opinion information may be a type of dementia, such as Alzheimer's disease, dementia with Lewy body, or vascular dementia.

Similarly, the opinion groups are not limited to the exemplary dementia progression group and the exemplary dementia non-progression group. The opinion groups may be groups according to types of dementia, such as an Alzheimer's disease group, a dementia-with-Lewy-body group, and the like.

The PACS server 11 may perform some or all of the functions of each of the processing units 45 to 49. For example, the normalization unit 46 may be included in the CPU of the PACS server 11, and the non-linear registration unit 47 and the dementia opinion derivation unit 48 may be included in the CPU 27 of the diagnosis support device 12.

The medical image is not limited to the head MRI image 15 in the example. The medical image may be a positron emission tomography (PET) image, a single photon emission computed tomography (SPECT) image, a computed tomography (CT) image, an endoscopic image, an ultrasound image, or the like.

The subject is not limited to the head in the example, and may be a chest, an abdomen, or the like. In addition, the disease is not limited to dementia in the example, and may be a heart disease, pneumonia, dyshepatia, or the like.

In each of the embodiments, for example, as a hardware structure of the processing unit that executes various processing, such as the RW control unit 45, the normalization unit 46, the non-linear registration unit 47, the dementia opinion derivation unit 48, the display control unit 49, and the local atrophy rate derivation unit 100, the following various processors may be used. The various processors include, as described above, the CPU 27 which is a general-purpose processor that functions as various processing units by executing software (an operation program 35), a programmable logic device (PLD) such as a field programmable gate array (FPGA) which is a processor capable of changing a circuit configuration after manufacture, a dedicated electric circuit such as an application specific integrated circuit (ASIC) which is a processor having a circuit configuration specifically designed to execute specific processing, and the like.

One processing unit may be configured by one of these various processors, or may be configured by a combination of two or more processors having the same type or different types (for example, a combination of a plurality of FPGAs and/or a combination of a CPU and an FPGA). Further, the plurality of processing units may be configured by one processor.

As an example in which the plurality of processing units are configured by one processor, firstly, as represented by a computer such as a client and a server, a form in which one processor is configured by a combination of one or more CPUs and software and the processor functions as the plurality of processing units may be adopted. Secondly, as represented by system on chip (SoC), there is a form in which a processor that realizes the functions of the entire system including a plurality of processing units with one integrated circuit (IC) chip is used. As described above, the various processing units are configured by using one or more various processors as a hardware structure.

Further, as the hardware structure of the various processors, more specifically, an electric circuit (circuitry) in which circuit elements such as semiconductor elements are combined may be used.

The technique of the present disclosure can also appropriately combine the various embodiments and/or the various modification examples. In addition, the technique of the present disclosure is not limited to each embodiment, and various configurations may be adopted without departing from the scope of the present disclosure. Further, the technique of the present disclosure extends to a program and a storage medium for non-temporarily storing the program.

The described contents and the illustrated contents are detailed explanations of a part according to the technique of the present disclosure, and are merely examples of the technique of the present disclosure. For example, the descriptions related to the configuration, the function, the operation, and the effect are descriptions related to examples of a configuration, a function, an operation, and an effect of a part according to the technique of the present disclosure. Therefore, it goes without saying that, in the described contents and illustrated contents, unnecessary parts may be deleted, new components may be added, or replacements may be made without departing from the spirit of the technique of the present disclosure. Further, in order to avoid complications and facilitate understanding of the part according to the technique of the present disclosure, in the described contents and illustrated contents, descriptions of technical knowledge and the like that do not require particular explanations to enable implementation of the technique of the present disclosure are omitted.

In this specification, "A and/or B" is synonymous with "at least one of A or B". That is, "A and/or B" means that only A may be included, that only B may be included, or that a combination of A and B may be included. Further, in this specification, even in a case where three or more matters are expressed by being connected using "and/or", the same concept as "A and/or B" is applied.

All documents, patent applications, and technical standards mentioned in this specification are incorporated herein by reference to the same extent as in a case where each document, each patent application, and each technical standard are specifically and individually described by being incorporated by reference.

## Claims

1. A diagnosis support device comprising:
a processor; and
a memory connected to or built in the processor,
wherein the processor is configured to
perform non-linear registration processing of a target image which is a medical image to be analyzed and at least one representative image generated from a plurality of medical images, and
input at least one of transformation amount information of the target image obtained by the non-linear registration processing or a feature amount derived from the transformation amount information to a disease opinion derivation model, and output a disease opinion from the disease opinion derivation model.

2. The diagnosis support device according to claim 1,
wherein the representative image is prepared for each of a plurality of attribute groups according to attributes of a patient.

3. The diagnosis support device according to claim 2,
wherein the attribute is at least one of age or gender.

4. The diagnosis support device according to claim 2 or 3,
wherein the representative image is prepared for at least one of a plurality of opinion groups obtained by further dividing the attribute groups according to the opinion.

5. The diagnosis support device according to claim 4,
wherein the opinion is content as to whether or not the disease is progressed, and
the opinion groups include a disease progression group in which the disease is progressed and a disease non-progression group in which the disease is not progressed.

6. The diagnosis support device according to any one of claims 1 to 5,
wherein the representative image is generated by repeatedly performing a series of processing, which includes processing of generating a plurality of registration images corresponding to the plurality of medical images by performing non-linear registration processing of the plurality of medical images and a first temporary representative image and processing of generating a second temporary representative image from the plurality of registration images, until an instruction to adopt the second temporary representative image as the representative image is input.

7. The diagnosis support device according to any one of claims 1 to 6,
wherein the medical image is an image in which a head of a patient appears, and
the disease opinion derivation model is a model that outputs, as the opinion, a dementia opinion on the patient.

8. The diagnosis support device according to any one of claims 1 to 7,
wherein the feature amount is a local atrophy rate derived by applying a Jacobi matrix to the transformation amount information.

9. An operation method of a diagnosis support device, the method comprising:
performing non-linear registration processing of a target image which is a medical image to be analyzed and at least one representative image generated from a plurality of medical images; and
inputting at least one of transformation amount information of the target image obtained by the non-linear registration processing or a feature amount derived from the transformation amount information to a disease opinion derivation model, and outputting a disease opinion from the disease opinion derivation model.

10. An operation program of a diagnosis support device, the program causing a computer to execute a process comprising:
performing non-linear registration processing of a target image which is a medical image to be analyzed and at least one representative image generated from a plurality of medical images; and
inputting at least one of transformation amount information of the target image obtained by the non-linear registration processing or a feature amount derived from the transformation amount information to a disease opinion derivation model, and outputting a disease opinion from the disease opinion derivation model.
